# EUROPÄISCHE PATENTSCHRIFT

(11) **EP 1 302 525 B1**
(45) Veröffentlichungstag und Bekanntmachung des Hinweises auf die Patenterteilung: **05.12.2007**
(21) Anmeldenummer: 02020725.4
(22) Anmeldetag: 14.09.2002
(51) Int. Cl.: C10G 49/00, C10G 45/08, C07C 5/02

(54) **Verfahren zur katalytischen Hydrierung**
Catalytic hydrogenation process
Procédé d' hydrogénation catalytique

(30) Priorität: 15.10.2001 DE 10150556
(43) Veröffentlichungstag der Anmeldung: 16.04.2003
(73) Patentinhaber: BASF Aktiengesellschaft, 67056 Ludwigshafen (DE)
(72) Erfinder: Koch, Michael, Dr., 67346 Speyer (DE); Schwab, Ekkehard, Dr., 67434 Neustadt (DE); Trübenbach, Peter, Dr., 67134 Birkenheide (DE); Schäfer, Harald, 67117 Limburghof (DE)

(56) Entgegenhaltungen:
- EP-A- 0 351 186
- EP-A- 0 922 687
- EP-A- 1 205 531
- US-A- 3 654 129

## Beschreibung

Die vorliegende Erfindung betrifft ein Verfahren zur katalytischen Hydrierung von Kohlenwasserstoffströmen. Insbesondere betrifft die Erfindung ein Verfahren zur katalytischen Hydrierung von Kohlenwasserstoffströmen ohne Verwendung eines vom zu hydrierenden Kohlenwasserstoffstrom verschiedenen Lösungsmittels.

In Raffinerien und petrochemischen Anlagen werden in großem Umfang Kohlenwasserstoffströme erzeugt, gelagert und verarbeitet. Ein typisches Verfahren ist das Steamcracken, bei dem Kohlenwasserstoffe wie Naphtha, Butan, Benzin oder LPG ("liquefied petroleum gas") thermisch gespalten werden und so olefinreiche Kohlenwasserstoffe erzeugt werden. Die meisten Steamcracker sind zur Maximierung des erzeugten Ethylens und/oder Propylens ausgelegt, wobei jedoch auch Kohlenwasserstoffe mit höherer Anzahl an C-Atomen erzeugt werden, deren Anteil mit ihrer Anzahl an C-Atomen deutlich abnimmt. Meistens werden die Bestandteile des sogenannten "C4-Schnitts" zumindest teilweise noch isoliert. Produkte mit mindestens 5 C-Atomen, der sogenannte "C5⁺-Schnitt" (bei Steamcrackern auch "Pyrolysebenzin" genannt) werden üblicherweise zunächst nicht weiter aufgetrennt. Pyrolysebenzin neigt zum Verharzen. Die dafür verantwortlichen reaktiven Alkine, Alkenine, Diene und/oder Polyene und/oder Alkin-, Alken-, Alkenin-, Dienund/oder Polyensubstituenten aromatischer Verbindungen werden daher üblicherweise zunächst selektiv katalytisch hydriert, um das Produkt zu stabilisieren. Anschließend wird aus dem selektiv hydrierten Strom meist durch Destillation ein Schnitt aus Verbindungen mit mindestens 6 Kohlenstoffatomen gewonnen. Dieser wird einer weiteren katalytischen Hydrierung zur Entfernung aller hydrierbaren Verbindungen, mit Ausnahme der aromatischen Ringgerüste ("aromatische Kerne") darin enthaltener aromatischer Verbindungen unterworfen. (Umgangssprachlich wird dieser zweite Hydrierschritt trotz des Erhalts der hydrierbaren Aromaten oft "Vollhydrierung" genannt, alternativ auch "Raffination"). Bei der Vollhydrierung, die meist in der Gasphase durchgeführt wird, werden gleichzeitig mit restlichen Alkinen, Alkeninen, Dienen und/oder Polyenen und/oder Alkin-, Alken-, Alkenin-, Dien- und/oder Polyensubstituenten aromatischer Verbindungen auch Alkene hydriert und/oder Heteroatome wie Schwefel, Stickstoff und/oder Sauerstoff enthaltende Verbindungen hydrierend gespalten, und so der Kohlenwasserstoffstrom auch von Heteroatome enthaltenden Verunreinigungen gereinigt, jedoch ohne in signifikantem Umfang die aromatischen Wertprodukte zu hydrieren. Der so erhaltene Kohlenwasserstoffstrom wird typischerweise einer Extraktivdestillation zur Gewinnung der darin enthaltenen Aromaten, insbesondere Benzol, Toluol und/oder Xylol, unterworfen. Die sonstigen Bestandteile des ursprünglichen Pyrolysebenzins werden anders verwertet, beispielsweise als Vergaserkraftstoff oder durch Rückführung in den Steamcracker. DE 1 067 160 offenbart ein Verfahren zur katalytischen Gasphasenhydrierung von selektiv hydriertem Pyrolysebenzin (als "Raffination" bezeichnet). In diesem Verfahren wird das selektiv hydrierte Pyrolysebenzin zusätzlich zur eigentlichen Gasphasenhydrierung einem weiteren Vorhydrierschritt in der Flüssigphase unterworfen, die Vollhydrierung also auf einen Vor- und einen Hauptreaktor aufgeteilt.

Ein anderes typisches Verfahren ist die Reformierung. Dabei werden im wesentlichen Alkane und/oder Cycloalkane enthaltende Kohlenwasserstoffe zu Aromaten reicheren Kohlenwasserstoffen umgewandelt. Dieses sogenannte Reformatbenzin enthält wie Pyrolysebenzin, aber in geringerem Umfang reaktive Alkine, Alkenine, Diene und/oder Polyene, so dass die hydrierende Entfernung dieser Verunreinigungen meist in einer einzigen Hydrierstufe durchgeführt wird. EP-A-792 928 (US-Äquivalent US 6,124,514) beschreibt ein Verfahren zur Extraktion von Benzol aus Reformatbenzin, das zuvor einer Hydrierung an Nickel- oder Palladiumkatalysatoren auf Aluminiumoxidträgern unterworfen wurde. WO-A-97/32944 (US-Äquivalent US 6,118,034) lehrt ein Verfahren zur Hydrierung von Reformatbenzin, das anschließend einer Aromatenextraktion zugeführt werden kann. Gemäß der Lehre von WO-A-97/32944 wird vorzugsweise der aus EP-A-672 452 (US-Äquivalent US 5,736,484) bekannte Nikkelkatalysator, der gemäß der Lehre von EP-A-672 452 unter anderen Verfahrensbedingungen zur Hydrierung aromatischer Verbindungen in Weißölen eingesetzt wird, zur Hydrierung ungesättigter unerwünschter Komponenten in Aromatenströmen unter Erhalt der Aromaten eingesetzt.

Eine weitere Quelle für Aromaten enthaltende Kohlenwasserstoffströme sind Kokereien, insbesondere das bei der Verkokung anfallende sogenannte Kokereirohbenzol. Ebenso fallen beim sogenannten Katcracken (meist das FCC-Verfahren, "fluid catalytic cracking") oder der Verschwelung fester Brennstoffe Aromaten enthaltende Kohlenwasserstoffströme an. All diesen Verfahren haben mit der Verarbeitung von Pyrolyse- oder Reformatbenzin gemein, dass die letztendlich als Wertprodukte zu isolierenden aromatischen Verbindungen mit unerwünschten hydrierbaren Verunreinigungen, in der Regel Alkine, Alkene, Alkenine, Diene, Polyene und/oder mit Alkin-, Alken-, Alkenin-, Dien- und/oder Polyenresten substituierte Aromaten und/oder Heteroatome wie Schwefel, Stickstoff und/oder Sauerstoff enthaltende Verbindungen, vermischt sind.

Üblicherweise werden auch bei diesen Kohlenwasserstoffströmen wie beim Pyrolyse- und Reformatbenzin diese Verunreinigungen vor Gewinnung der aromatischen Wertprodukte durch katalytische Hydrierung entfernt.

Ein Verfahren, bei dem das Wertprodukt keine aromatische Verbindung ist, ist beispielsweise die Oligomerisierung niedermolekularen Alkene, beispielsweise die Di-/Trimerisierung von C4-Verbindungen wie iso-Buten zu dem üblicherweise als "iso-Okten" bezeichneten Gemisch von Di-iso-butylen, meist kleineren Anteilen von Tri-iso-butylen und meist größeren Anteilen der entsprechenden gesättigten Kohlenwasserstoffe, und die nachfolgende katalytische Hydrierung der Oligomere, im Falle von iso-Okten zu dem üblicherweise als "iso-Oktan" bezeichneten entsprechenden Gemisch vollständig gesättigter Kohlenwasserstoffe. Derartige Oligomere sind wegen ihrer hohen Research- und Motor-Oktanzahlen wertvolle Motorkraftstoffkomponenten. So offenbart EP-A-989 106 ein integriertes Verfahren zur Herstellung gesättigter Oligomerer durch Oligomerisierung kurzkettiger Olefine und anschließende Hydrierung. WO-A-99/26905 und EP-A-994 088 lehren ähnliche Verfahren zur Herstellung gesättiger Dimerer oder Oligomerer. EP-A-881 275 (US-Äquivalent US 6,037,510) beschreibt ein Verfahren zur katalytischen Hydrierung von iso-Okten zu iso-Oktan.

EP-A-922 687 (US-Äquivalent US 6,096,931) offenbart ein Verfahren zur katalytischen Gasphasenhydrierung von Aldehyden zu Alkoholen, bei dem dem zu hydrierenden Aldehyd stickstoffhaltige Basen zugesetzt werden, um die Bildung von Nebenprodukten wie Alkanen (durch Decarbonylierung), Ethern und/oder Estern aus den Aldehyden zu vermeiden.

EP-A-135 442 (US-Äquivalent US 4,571,442) lehrt ein Verfahren zur Hydrierung von Acetylen in einem C2-Schnitt an einem Palladiumkatalysator, bei dem die Hydrierung in einem Lösungsmittel in Gegenwart eines Amins durchgeführt wird. Nach der Hydrierung wird der C2-Schnitt vom Lösungsmittel getrennt und letzteres wieder in den Reaktor zurückgeführt. EP-A-151 356 (US-Äquivalent US 4,587,369) lehrt ein ähnliches Verfahren zur Hydrierung eines C4-Schnitts unter Verwendung einem aminhaltigen Lösungsmittels. Die Abtrennung, Reinigung und Rückführung eines Lösungsmittels ist jedoch aufwendig und wirtschaftlich nachteilig.

EP-A-0 351 186 offenbart die Verwendung von Aminer als Inhibitoren der Koksbildung in katalytischen Hydrierungsreaktoren.

Derartige katalytische Hydrierungen werden in der Gasphase, in der gemischten Gas-/Flüssigphase oder in der Flüssigphase durchgeführt. Bei Durchführung in der Gasphase werden etwaige nicht verdampfbare Anteile vor dem katalytischen Reaktor bei der Verdampfung automatisch abgetrennt und können keine Ablagerungen auf dem Katalysator bilden. Allerdings entsteht bei derartigen Hydrierungen aus den zu hydrierenden reaktiven Verbindungen in gewissem Umfang stets ein hochsiedendes oder nicht verdampfbares Gemisch aus Oligomeren und Polymeren dieser Verbindungen. Dieses lagert sich auf dem Katalysator und in nachgeschalteten Anlagenteilen ab, seine Bildung ist daher unerwünscht und zu minimieren. Bei Hydrierungen in Gegenwart flüssiger Phase wird auf dem Katalysator oder im Reaktor keine derartige Ablagerung beobachtet, da entstehende Oligo- und Polymere in der Flüssigkeit gelöst und so vom Katalysator und von Anlagenteilen abgewaschen und abgeführt werden. In welchem Ausmaß diese hochsiedenden oder nicht verdampfbaren Bestandteile des Reaktionsprodukts in nachgeschalteten Verfahren stören, hängt zwar von diesen nachgeschalteten Verfahren ab, im Allgemeinen wird jedoch stets ein möglichst niedriger Oligo- und/oder Polymerengehalt angestrebt. In manchen Fällen können auch durch Spaltreaktionen am Katalyastor unerwünschte Spaltprodukte entstehen, die - wenn sie nicht auch andere unerwünschte Eigenschaften haben - zumindest zu einer Ausbeuteerniedrigung führen.

Es stellt sich daher die Aufgabe, ein Verfahren zur katalytischen Hydrierung von Kohlenwasserstoffströmen zu finden, bei dem möglichst wenig unerwünschte Nebenkomponenten wie Oligo- und/oder Polymere und/oder Spaltprodukte entstehen. Dementsprechend wurde ein Verfahren zur katalytischen Hydrierung von Kohlenwasserstoffströmen ohne Verwendung eines vom zu hydrierenden Kohlenwasserstoffstrom verschiedenen Lösungsmittels gefunden, gemäß Anspruch 1.

Mit dem erfindungsgemäßen Verfahren wird die Bildung unerwünschter Nebenkomponenten weitgehend vermieden, ohne dass ein Selektivitätsverlust des Katalysators beobachtet wird. Die Katalysatorstandzeiten bei Gasphasenhydrierungen werden erheblich verlängert, und bei Flüssigphasenhydrierungen treten keine durch Hochsieder oder nicht verdampfbare Substanzen bedingten Probleme in nachgeschalteten Verfahren auf, oder sie werden erheblich verringert. Das erfindungsgemäße Verfahren kommt dennoch ohne andere Nachteile bekannter Verfahren wie etwa den mit einer Lösungsmittelabtrennung, -aufarbeitung und -rückführung verbundenen Aufwand aus. Zusätzlich wurde gefunden, dass bei den Verfahren, bei denen ein Verlust an hydrierbaren Wertprodukten wie etwa Aromaten durch mangelnde Selektivität der Hydrierung der zu hydrierenden, im Wertproduktstrom unerwünschten Komponenten auftreten kann, dieser Wertproduktverlust mit dem erfindungsgemäßen Verfahren deutlich verringert wird.

Im erfindungsgemäßen Verfahren wird dem Einsatzstoffstrom ein Trialkylamin zugesetzt. T

Unter den Trialkylamine werden Trimethylamin und Triethylamin bevorzugt. In besonders bevorzugter Weise wird Triethylamin verwendet.

Das Trialkylamin wird dem Einsatzstoffstrom zugesetzt. Dies kann vor dem Katalysatorbett, in einer oder mehreren Zuführungsstellen, oder auch zumindest partiell an einer oder mehreren Stellen im Katalysatorbett erfolgen. Das Trialkylamin wird in gasförmigem oder flüssigen Zustand zugesetzt, sie kann auch in dem Einsatzstoff des erfindungsgemäßen Verfahrens gelöst zugesetzt werden.

Das Trialkylamin wird in einer Menge zugesetzt, die zur Erzielung des gewünschten Effekts der Verringerung der Bildung unerwünschter Nebenkomponenten ausreicht, aber nicht so groß ist, dass sie bei der Weiterverarbeitung des hydrierten Produkts nicht stört. Dies ist von dieser Art der Weiterverarbeitung abhängig und in wenigen Routineversuchen leicht zu ermitteln. Im Allgemeinen wird die basische Verbindung in einer Menge von mindestens 0,1 Gew.-ppm, jeweils bezogen auf den gesamten Einsatzstoffstrom, zugesetzt, vorzugsweise in einer Menge von mindestens 1 Gew.-ppm und in besonders bevorzugter Weise in einer Menge von mindestens 2 Gew.-ppm, beispielsweise auch in einer Menge von mindestens 10 Gew.-ppm. Die zugesetzte Menge an basischer Verbindung beträgt ferner im Allgemeinen höchstens 10 000 Gew.-ppm, vorzugsweise höchstens 1 000 Gew.-ppm und in besonders bevorzugter Weise höchstens 100 Gew.-ppm.

Aromaten enthaltende Kohlenwasserstoffströme, die dem erfindungsgemäßen Verfahren unterworfen werden, sind (wahlweise selektiv vorhydriertes) Pyrolysebenzin, Reformatbenzin und Kokereirohbenzol.

Derartige Hydrierverfahren sind - ohne den erfindungsgemäßen Zusatz eines Trialkylamins - wohlbekannt. Die Wahl der Art der Durchführung des erfindungsgemäßen Verfahrens (Gasphase, gemischte Gas-/Flüssigphase, Flüssigphase) und die Wahl der Hydrierparameter (Rückführungen, Temperaturen, Drücke, Raumgeschwindigkeiten und sonstige Parameter) ist eine Routineaufgabe des Fachmanns und erfolgt je nach der gestellten Hydrieraufgabe wie bei derartigen Hydrierverfahren allgemein üblich.

Typische Verfahrensparameter für Flüssigphasenverfahren sind ein Druck im Bereich von 1 bis 20 bar, eine Temperatur im Bereich von 20 bis 200 °C, eine Raumgeschwindigkeit ("WHSV") bei Flüssigphasenverfahren im Bereich von 1 bis 10 kg Einsatzstoffstrom pro Liter Katalysator und Stunde und bei Gasphasenverfahren im Bereich von 0,3 bis 5 kg Einsatzstoffstrom pro Liter Katalysator und Stunde, ein molares Verhältnis von Wasserstoff zu den zu hydrierenden Bindungen im Bereich von 1 bis 100, und ein Rücklaufverhältnis (vor den Reaktor zurückgeführtes Produkt zu erstmals dem Reaktor zugeführten Frisch-Einsatzstoff) im Bereich von 0 bis 50.

Es ist möglich, dass beim erfindungsgemäßen Verfahren, verglichen mit einem entsprechenden bekannten Verfahren, das mit Ausnahme des Aminzusatzes auf identische Weise durchgeführt wird, ein geringer Rückgang des Umsatzes pro Reaktordurchgang auftritt. Dies kann mit bekannten Maßnahmen zur Umsatzerhöhung, insbesondere Temperaturerhöhung, Erhöhung des Rücklaufverhältnisses oder anderweitige Verringerung des Reaktordurchsatzes, oder Verwendung etwas größerer Katalysatorvolumina kompensiert werden, falls nötig. Im allgemeinen wird der Nachteil eines verringerten Umsatzes, falls er auftritt, durch die Verringerung der Bildung unerwünschter hochsiedender oder nicht verdampfbarer Verbindungen und meist auch durch die mit dem erfindungsgemäßen Verfahren erreichte höhere Selektivität der Hydrierung wirtschaftlich kompensiert oder überkompensiert.

Im erfindungsgemäßen Verfahren kann jeder für die Hydrierung von Kohlenwasserstoffströmen bekannte Hydrierkatalysator verwendet werden. Im Allgemeinen enthalten diese Katalysatoren mindestens ein Element aus den Gruppen 9 (Kobalt, Rhodium, Iridium) und 10 (Nickel, Palladium und Platin) des Periodensystems der Elemente und wahlweise auch ein Element aus der Gruppe 6 (Chrom, Molybdän, Wolfram) des Periodensystems der Elemente auf einem Katalysatorträger.

Der Katalysatorträger ist im Allgemeinen ein üblicher oxidischer Katalysatorträger wie beispielsweise Siliciumdioxid, Aluminiumoxid, Zirkondioxid oder ein Gemisch dieser Oxide.

Ein geeigneter Katalysator enthält beispielsweise Palladium und/oder Platin auf Aluminiumoxid. Bevorzugterweise wird jedoch ein Katalysator verwendet, der Nickel und/oder Kobalt auf einem Katalysatorträger enthält. Wahlweise enthält dieser Katalysator auch Molybdän. In besonders bevorzugter Weise enthält der im erfindungsgemäßen Verfahren verwendete Katalysator Nickel oder Nickel und Molybdän oder Kobalt und Molybdän, jeweils auf einem Katalysatorträger. Meist wird für die Hydrierung von Reformatströmen oder ungesättigten Alkendimeren oder -oligomeren ein Nickel enthaltender Katalysator, für die Vollhydrierung von Pyrolysebenzin oder Kokereirohbenzol ein Kobalt und Molybdän oder ein Nickel und Molybdän enthaltender Katalysator verwendet.

Derartige Katalysatoren sind bekannt und handelsüblich. Beispielsweise sind derartige Katalysatoren unter den Bezeichnungen "H0-22" (Palladium auf Katalysatorträger), "H1-89" (Nickel auf Katalysatorträger), "M8-12" (Kobalt/Molybdän auf Katalysatorträger) oder "M8-21" (Nickel/Molybdän auf Katalysatorträger) von BASF Aktiengesellschaft, Ludwigshafen, Deutschland, erhältlich. Der in EP-A-672 452 offenbarte Katalysator ist ebenso für das erfindungsgemäße Verfahren geeignet.

### Beispiele

Die Versuche zur Gasphasenhydrierung wurden in einem kontinuierlich betriebenen Festbettreaktor (50 ml Katalysatorvolumen) mit Kreisgasführung durchgeführt. Der Einsatzstoffstrom und gegebenenfalls das Amin wurden mit Hilfe einer HPLC-Pumpe über einen Vorheizer in den Reaktor dosiert. Das hydrierte Produkt wurde in einem Abscheider abgetrennt und über einen mit Natronlauge gefüllten Waschturm vom durch hydrierende Spaltung enthaltener Schwefelverbindungen gebildeten Schwefelwasserstoff befreit.

Die Versuche zur Flüssigphasenhydrierung wurden in einem kontinuierlich betriebenen Festbettreaktor (50 ml Katalysatorvolumen) in Rieselfahrweise ohne Rückführung von hydriertem Produkt vor den Reaktor betrieben. Das hydrierte Produkt wurde in einem Abscheider abgetrennt.

### Beispiel 1

### Hydrierung eines Reformatbenzins in der Flüssigphase unter Zusatz von Triethylamin

Bei 60 °C, 4 bar Druck, einer WHSV von 6 kg Einsatzstoff / Liter Katalysator und Stunde sowie einem Verhältnis von Wasserstoff zur Summe von Olefinen und Diolefinen von 3 Mol/Mol wurde ein Reformatbenzin (ca. 35 Gew.-% Benzol, 3 Gew.-% Olefinen und 300 Gew.ppm MCPD (Akronym von Methylcyclopentadien) an einem gemäß Beispiel 1 der EP-A-672 452 hergestellten Nickelkatalysator auf Träger hydriert. Die Hydrierung wurde zunächst ohne Zudosierung von Amin betrieben, bis der stationäre Zustand erreicht war, was in diesem Versuch nach 220 Stunden Betriebsdauer der Fall war. Anschließend wurden über 300 Stunden 300 Gew.-ppm Triethylamin, daran anschließend über weitere 300 Stunden Betriebszeit 10 ppm Triethylamin und über die anschließenden 100 Stunden Betriebszeit 2 ppm Triethylamin zugesetzt, bis wieder ein stationärer Zustand erreicht war. In diesem Versuch wurde dieser stationäre Zustand nach insgesamt 900 Stunden Versuchsdauer, ab Beginn des Versuchs gerechnet, erreicht.

Die jeweils im stationären Zustand ermittelten Ergebnisse sind in Tabelle 1 gegenübergestellt.

In den folgenden Tabellen ist die ab Beginn des Versuchs gerechnete Laufzeit bis zum Erreichen der jeweiligen stationären Zustände im Kopf der betreffenden Spalten angegeben.

**Tabelle 1**

| | ohne Amin (220 h) | mit Amin (900 h) |
|---|---|---|
| MCPD-Umsatz [Mol-%] | 100 | 100 |
| Olefinumsatz [Mol-%] | 99,9 | 92 |
| Benzolumsatz [Mol-%] | 6,4 | < 0,2 |
| Oligomere [Gew.-ppm] | 32 | < 5 |

Der Vergleich in Tabelle 1 zeigt, dass beim erfindungsgemäßen Verfahren, verglichen mit konventioneller Vorgehensweise, bei nur leicht gesunkenem Olefinumsatz der unerwünschte Wertproduktverlust durch Benzolhydrierung und die Bildung von Oligomeren deutlich zurückgehen.

### Beispiel 2

### Hydrierung eines Reformatbenzins in der Flüssigphase unter Zusatz von Trimethylamin

Beispiel 1 wurde wiederholt, wobei jedoch ein Reformatbenzin mit ca. 60 Gew.-% Benzol, 2 Gew.-% Olefinen und 500 Gew.-ppm MCPD verwendet und Trimethylamin statt Triethylamin zugesetzt wurde.

Die jeweils im stationären Zustand ermittelten Ergebnisse sind in Tabelle 2 gegenübergestellt.

**Tabelle 2**

| | ohne Amin (120 h) | mit Amin (750 h) |
|---|---|---|
| MCPD-Umsatz [Mol-%] | 100 | 100 |
| Olefinumsatz [Mol-%] | 99,9 | 94 |
| Benzolumsatz [Mol-%] | 7,4 | 0,6 |
| Oligomere [Gew.-ppm] | 40 | < 5 |

Der Vergleich in Tabelle 2 zeigt, dass beim erfindungsgemäßen Verfahren, verglichen mit konventioneller Vorgehensweise, bei nur leicht gesunkenem Olefinumsatz der unerwünschte Wertproduktverlust durch Benzolhydrierung und die Bildung von Oligomeren deutlich zurückgehen.

### Beispiel 3

### Hydrierung eines Reformatbenzins in der Flüssigphase unter Zusatz von Ammoniak

Beispiel 1 wurde wiederholt, wobei jedoch ein Reformatbenzin mit ca. 40 Gew.-% Benzol, 3 Gew.-% Olefinen und 500 Gew.-ppm MCPD verwendet und nach Erreichen des stationären Zustands ohne Amindosierung 100 Gew.-ppm Ammoniak statt Triethylamin zugesetzt wurde.

Die jeweils im stationären Zustand ermittelten Ergebnisse sind in Tabelle 3 gegenübergestellt.

**Tabelle 3**

| | ohne Amin (190 h) | mit Amin (310 h) |
|---|---|---|
| MCPD-Umsatz [Mol-%] | 100 | 70 |
| Olefinumsatz [Mol-%] | 99,8 | 20 |
| Benzolumsatz [Mol-%] | 5,2 | 0 |
| Oligomere [Gew.-ppm] | 80 | 20 |

Der Vergleich in Tabelle 3 zeigt, dass beim erfindungsgemäßen Verfahren, verglichen mit konventioneller Vorgehensweise, der unerwünschte Wertproduktverlust durch Benzolhydrierung und die Bildung von Oligomeren deutlich zurückgehen, dass aber die Verwendung von Ammoniak auch zu einem - verglichen mit anderen Aminen - höheren Ausbeuterückgang führt.

### Beispiel 4

### Vollhydrierung eines selektiv vorhydrierten Pyrolysebenzins in der Gasphase unter Zusatz von Triethylamin

Bei 320 °C, 30 bar Druck, einer WHSV von 1,6 kg Einsatzstoff /Liter Katalysator und Stunde, einem Verhältnis von Kreisgas zu erstmals dem Reaktor zugeführtem Einsatzstoff von 400 Nl/kg (Nl Kreisgas je kg frischer Einsatzstoff; Nl: "Normliter", Volumen bei 1 bar Druck abs. und einer Temperatur von 0°C) wurde ein selektiv vorhydriertes Pyrolysebenzin (ca. 35 Gew.-% Benzol, 20 Gew.-% Toluol, 8 Gew.-% Xylole, Bromzahl (ASTM D1159) 27 g/100 g und 300 ppm Schwefel (als Bestandteil von Schwefelverbindungen)) an einem handelsüblichen Kobalt/Molybdän-Katalysator auf Aluminiumoxidträger (Katalysator M8-12 der BASF Aktiengesellschaft, 14,5 Gew.-% MoO₃, 3,4 Gew.-% CoO, BET-Oberfläche 240 m²/g, in Form von Stränglingen mit 2,5 mm Durchmesser) hydriert, bis der stationäre Zustand erreicht war. Anschließend wurden 100 Gew.-ppm Triethylamin zugesetzt, bis wiederum der stationäre Zustand erreicht war.

Die jeweils im stationären Zustand ermittelten Ergebnisse sind in Tabelle 4 gegenübergestellt.

**Tabelle 4**

| | ohne Amin (120 h) | mit Amin (320 h) |
|---|---|---|
| Bromzahl [g/100 g] | 0,5 | 0,5 |
| Schwefel [ppm] | 2 | 1 |
| Benzolumsatz [Mol-%] | 0,7 | 0,66 |
| Cyclohexylbenzol [Gew.-ppm] | 140 | 60 |
| Hochsieder¹ [Gew.-ppm] | 1500 | 1000 |

| | | |
|---|---|---|
| 1 als "Hochsieder" werden Verbindungen mit einer Retentionszeit von 74-100 min bei Aufnahme eines Gaschromatogramms mit einer Petrocol-Säule bezeichnet | | |

Der Vergleich in Tabelle 4 zeigt, dass beim erfindungsgemäßen Verfahren, verglichen mit konventioneller Vorgehensweise, bei identischem Olefinumsatz (gleiche Bromzahl) der unerwünschte Wertproduktverlust durch Benzolhydrierung und die Bildung von Oligomeren deutlich zurückgehen.

### Beispiel (nicht erfindungsgemäß)

### Hydrierung eines iso-Oktens in der Gasphase unter Zusatz von Triethylamin

Bei 22 bar Druck, einer WHSV von 2,8 kg Einsatzstoff / Liter Katalysator und Stunde (entspricht in diesem Fall einer WHSV von 1,1 kg Olefinanteil im Einsatzstoffstrom / Liter Katalysator und Stunde) sowie einem Verhältnis von Kreisgas zu erstmals dem Reaktor zugeführtem Einsatzstoff von 3 200 Nl/kg wurde ein iso-Okten-Gemisch (34,2 Gew.-% Di-iso-butylen, 3,8 Gew.-% Tri-iso-butylen, 62 Gew.-% iso-Oktan und 52 Gew.-ppm Thiophen (entspricht 20 Gew.ppm Schwefel)) an einem handelsüblichen Kobalt/Molybdän-Katalysator auf Aluminiumoxidträger (Katalysator M8-12 der BASF Aktiengesellschaft, 14,5 Gew.-% MoO₃, 3,4 Gew.-% CoO, BET-Oberfläche 240 m²/g, in Form von Stränglingen mit 2,5 mm Durchmesser) bei 270 °C hydriert, bis der stationäre Zustand erreicht war. Anschließend wurde die Temperatur auf 250 °C gesenkt, und wiederum der stationäre Zustand abgewartet. Anschließend wurden bei ansonsten konstanten Bedingungen 100 Gew.-ppm Triethylamin zugesetzt, bis wiederum der stationäre Zustand erreicht war, und danach anschließend wurde die Temperatur bei ansonsten konstanten Bedingungen auf 270 °C erhöht, wiederum bis zum Erreichen des stationären Zustands.

Die jeweils im stationären Zustand ermittelten Ergebnisse sind in Tabelle 5 gegenübergestellt.

**Tabelle 5**

| | ohne Amin | | mit Amin | |
|---|---|---|---|---|
| | 270 °C (70 h) | 250 °C (170 h) | 250 °C (370 h) | 270 °C (470 h) |
| Olefinumsatz [Mol-%] | 99,1 | 99,0 | 97,2 | 98,9 |
| Selektivität C8+ [Mol-%] | 97,0 | 97,6 | 99,3 | 98,6 |
| Bromzahl [g/100 g] | 0,2 | 0,8 | 1,9 | 0,4 |
| Schwefel [Gew.-ppm] | 1 | 1 | <1 | <1 |

Der Vergleich in Tabelle 5 zeigt, dass beim erfindungsgemäßen Verfahren, verglichen mit konventioneller Vorgehensweise, bei nur wenig gesunkenem Olefinumsatz die Selektivität der Hydrierung steigt.

## Patentansprüche

1. Verfahren zur katalytischen Hydrierung von Kohlenwasserstoffströmen, ausgewählt aus der Gruppe, bestehend aus Pyrolysebenzin, Reformatbenzin und Kokereirohbenzol ohne Verwendung eines vom zu hydrierenden Kohlenwasserstoffstrom verschiedenen Lösungsmittels, **dadurch gekennzeichnet, dass** man dem Einsatzstoffstrom ein Trialkylamin zusetzt.

2. Verfahren nach Anspruch 1, **dadurch gekennzeichnet, dass** man als zu hydrierenden Kohlenwasserstoffstrom Reformatbenzin einsetzt.

3. Verfahren nach Anspruch 1, **dadurch gekennzeichnet, dass** man Triethylamin zusetzt.

4. Verfahren nach Anspruch 1, **dadurch gekennzeichnet, dass** man einen Katalysator verwendet, der mindestens ein Element aus den Gruppen 9 und 10 des Periodensystems der Elemente und wahlweise auch ein Element aus der Gruppe 6 des Periodensystems der Elemente auf einen Katalysatorträger enthält.

5. Verfahren nach Anspruch 4, **dadurch gekennzeichnet, dass** man einen Nickel oder Kobalt, und wahlweise auch Molybdän, enthaltenden Katalysator auf einem Siliciumdioxid-, Zirkondioxid- und/oder Aluminiumoxidträger verwendet.

## Claims

1. A process for the catalytic hydrogenation of hydrocarbon streams selected from the group consisting of pyrolysis gasoline, reformed gasoline and cooking-oven benzol without using a different solvent from the hydrocarbon stream to be hydrogenated, which comprises adding a trialkylamine to the starting-material stream.

2. The process according to claim 1, wherein reformed gasoline is used as the hydrocarbon stream to be hydrogenated.

3. The process according to claim 1, wherein triethylamine is added.

4. The process as claimed in claim 1, wherein use is made of a catalyst which comprises at least one element from groups 9 and 10 of the Periodic Table of the Elements and optionally also an element from group 6 of the Periodic Table of the Elements on a catalyst support.

5. The process as claimed in claim 4, wherein use is made of a catalyst containing nickel or cobalt, and optionally also molybdenum, on a silicon dioxide, zirconium dioxide and/or aluminum oxide support.

## Revendications

1. Procédé d'hydrogénation catalytique de courants d'hydrocarbures choisis dans le groupe constitué de l'essence de pyrolyse, de l'essence de reformat et du benzène brut de cokerie, sans utiliser un solvant différent du courant d'hydrocarbure que l'on souhaite hydrogéner, **caractérisé en ce que** l'on ajoute une trialkylamine au courant de substance utilisé.

2. Procédé selon la revendication 1, **caractérisé en ce que** l'on utilise de l'essence de reformat comme courant d'hydrocarbure que l'on souhaite hydrogéner.

3. Procédé selon la revendication 1, **caractérisé en ce que** l'on ajoute une triéthylamine.

4. Procédé selon la revendication 1, **caractérisé en ce que** l'on utilise un catalyseur qui contient au moins un élément issu des groupes 9 et 10 du système périodique des éléments et, au choix, également un élément issu du groupe 6 du système périodique des éléments, sur un support de catalyseur.

5. Procédé selon la revendication 4, **caractérisé en ce que** l'on utilise un catalyseur contenant du nickel ou du cobalt et, au choix, également du molybdène, sur un support de dioxyde de silicium, de dioxyde de zirconium et/ou de d'oxyde d'aluminium.
